(19) Europäisches Patentamt
European Patent Office
Office européen des brevets

(11) Publication number: **0 200 212 B1**

# (12) EUROPEAN PATENT SPECIFICATION

(45) Date of publication of patent specification: **11.08.93**

(51) Int. Cl.$^5$: **C07C 319/00**, C07C 323/00

(21) Application number: **86105924.4**

(22) Date of filing: **29.04.86**

Divisional application 91117421.7 filed on 29/04/86.

## (54) Process for producing polythiobisphenols and process for producing mercaptophenols by the hydrogenolysis of the same.

(30) Priority: **30.04.85 JP 94045/85**

(43) Date of publication of application:
**05.11.86 Bulletin 86/45**

(45) Publication of the grant of the patent:
**11.08.93 Bulletin 93/32**

(84) Designated Contracting States:
**DE FR GB**

(56) References cited:
**DE-B- 2 225 278**
**GB-A- 1 153 269**
**GB-A- 1 425 278**

(73) Proprietor: **SUMITOMO SEIKA CHEMICALS CO., LTD.**
**346-1, Miyanishi Harimacho, Kako-gun**
**Hyogo-ken(JP)**

(72) Inventor: **Onoe, Akira**
**61-7, Honmaci 1-chome Hirohata-ku**
**Himeji Hyogo(JP)**
Inventor: **Kawamura, Masao**
**18-10, Higashi-Asagirioka**
**Akashi Hyogo(JP)**
Inventor: **Nishi, Tadaaki**
**11-7, Shin-Kanno 8-chome**
**Kakogawa Hyogo(JP)**
Inventor: **Kobayashi, Kenzo**
**905-104 Futamata-Ikenouchi Hiraoka-cho**
**Kakogawa Hyogo(JP)**
Inventor: **Yoshikawa, Masato**
**790-5, Kuchiri Onoe-cho**
**Kakogawa Hyogo(JP)**

(74) Representative: **von Kreisler, Alek, Dipl.-Chem. et al**
**Patentanwälte, Von Kreisler-Selting-Werner,**
**Postfach 10 22 41, Bahnhofsvorplatz 1**
**W-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to a process for producing polythiobisphenols.

Polythiobisphenols are now in use as highly reactive sterlizers as well as intermediates for the production of mercaptophenols which are in use as intermediates for the production of medical supplies, agricultural pesticides, color developers and others, or as antioxidants for rubbers and plastics.

It is already known that the reaction of phenols with sulfur monochloride (disulfur dichloride, $S_2Cl_2$) in a solvent to provide dithiobisphenols, and a variety of solvents are claimed to be suitable for use, for example, benzene (Z. S. Ariyan et al., J. Chem. Soc., 3876 (1962), carbon tetrachloride (E. B. Hotelling, J. Org. Chem., 24, 1598 (1959), petroleum ethers (German Patent No. 1,145,630), and organic polar solvents having solubility to water of not less than 20 (Japanese Patent Disclosure No. 50-24233). Further according to Ariyan et al. anhydrous hydrogen chloride, ferric chloride, stannic chloride, etc., are effective to accelerate the reaction.

However, these methods have been found to have substantial disadvantages. For example, the methods of Ariyan et al. and of Hotelling produce a large amount of monothiobisphenols and sulfur as byproducts in addition to dithiobisphenols. The use of the Lewis acid as a catalyst as above leaves large portions of the phenol used unreacted, and hence the yield of polythiobisphenol is very low. The catalysis of anhydrous hydrochloric acid is not feasible for industrial production of polythiobisphenols.

It is also disclosed (British Patent No. 1,345,311) that the reaction of phenols with sulfur monochloride in N,N-dimethylformamide or N-methylpyrolidone as a solvent in the absence of hydrogen sulfide and a catalyst provides polythiobisphenols in improved yields of about 60-90 %. However, according to the prior art, the alkylated acid amides participate in the reaction as an acceptor of the hydrochloric acid generated in the reaction, so that the alkylated acid amide must be used in such amounts that the amide has therein nitrogen atoms equivalent to or more than moles of the phenol used. That is, the alkylated acid aside equivalent to the hydrochloric acid generated in the reaction is converted to the acid amide hydrochloride in the reaction. In fact, the prior process typically uses as much as at least 400 ml of the acid amide per mole of the phenol used, to obtain the corresponding polythiobisphenol in the yield as above stated. Therefore, in this prior method, it is necessary to make the acid aside hydrochloride neutral with an alkali, for example, sodium hydroxide, after the reaction. This results in the production of a large amount of water, and the removal of the water from the reaction mixture is difficult but also deteriorates the process economy. When ammonia is used as an alkali instead of sodium hydroxide, a large amount of ammonium chloride is produced by the neutralization of the acid amide hydrochloride. This makes difficult the recovery and the reuse of the acid amide.

The prior process has further disadvantages in that the process involves the rather violent neutralization reaction of the acid amide in large amounts and hydrochloric acid generated, and accordingly the reaction generates great amounts of neutralization heat. The higher the reaction temperature is, the sore vigorous the reaction proceeds, and results in the production of undesired byproducts such as p-chlorophenol and bis(4-hydroxyphenyl)sulfide. Therefore, it is neccessary that the reaction is carried out at a low temperature as low as -10 °C to -40 °C to obtain the polythiobisphenol in the yields as called for in the literature, and the process needs a large cooling cost.

The present inventors have therefore made extensive studies on processes for producing polythiobisphenols, and found out that the specified catalysts accelerate the reaction of phenols with sulfur monochloride in a specific organic polar solvent to provide polythiobisphenols in high yields with a minimum production of undesired monothiobisphenols.

Therefore, an object of the present invention is to provide a process for producing polythiobisphenols by the reaction of phenols with sulfur monochloride in the presence of a novel catalyst.

A still further object of the present invention is to provide a process for producing polythiobisphenols which is simple in operation and mild in reaction conditions so as to be suitable for industrial production of polythiobisphenols.

According to the present invention, a process for producing polythiobisphenols is provided according to claim 1.

Further according to the present invention, another process for producing polythiobisphenols is provided, the process comprising:

reacting the phenol as above with sulfur monochloride in a polar organic solvent in the presence of bromine or an alkali metal halide, in amounts of 200-2000 ppm based on the amount of sulfurmonochloride.

At first, the process for production of polythiobisphenols according to the invention is described.

The phenol used as the starting material in the process of the invention is represented by the general formula

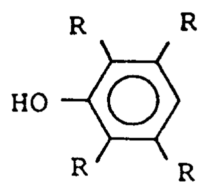

(I)

or

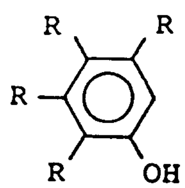

(II)

That is, the phenol should have no substituent at least at the para position or at least at one of the ortho positions to the hydroxyl group, since a first phenol is united with a second phenol at the para or ortho positions to the hydroxyl via polysulfide linkage. The substituents R's are independently hydrogens, halogens or alkyls which have preferably 1 to 4 carbons. Therefore, the phenol preferably used includes, for example, phenol, o-cresol, o-chlorophenol, 2,6-dimethylphenol, 2,6-diisopropylphenol, 2,6-di-tertiary butylphenol, 2,6-dichlorophenol, 2,3,5,6-tetramethylphenol, p-cresol, p-isopropylphenol, p-tertiary butylphenol, p-chlorophenol and p-fluorophenol.

The reaction of the phenol with sulfur monochloride is carried out in a polar organic solvent selected from the group consisting of aliphatic ethers, alicyclic ethers, alkyleneglycol dialkylethers, polyalkyleneglycol dialkylethers, alkyleneglycol monoether monoester, polyalkyleneglycol monoether monoester and aliphatic carboxylic acid alkylesters. In general, the more polar a solvent is, the higher the selectivity of the reaction and the yield of polythiobisphenol are. The most preferred solvents are, for example, diethylether, dioxane, dimethoxyethane, ethylglycol acetate (ethyleneglycol monoethylether monoacetate), ethyl acetate and the like. The solvent is normally used in amounts of 2-10 times as much as the weight of the phenol used, and preferably 3-8 times as much as the amount of the phenol used.

The reaction according to the invention is carried out in the presence of a catalyst. The first group of the catalyst is a nitrogen-containing organic compound which is selected from the group consisting of tertiary amines, preferably aliphatic and alicyclic tertiary amines, quaternary ammoniums, alkylated acid amides and heteroaromatic compounds. Preferred catalysts are, for example, tertiary amines such as trimethylamine, triethylamine, triethylenediamine or tetramethylethylenediamine, organic quaternary ammoniums such as trialkylarylammonium halide, e.g., trimethylbenzylammonium chloride and triethylbenzylammonium chloride or tetraalkylammonium halide, e.g. tetramethylammonium chloride, alkylated acid amides such as N,N-dialkylformamide, e.g., N,N-dimethylformamide, N,N-dialkylacetamide, e.g., N,N-dimethylacetamide or N-alkylpyrolidone, e.g., N-methylpyrolidone, and heteroaromatic compounds such as pyridine, 2-chloropyridine, $\alpha$ -picoline or $\beta$ -picoline.

The above catalyst is normally used in amounts of 1-30 % by weight based on the amount of the phenol used. The use of less than 1 % by weight based on the phenol used is lacking in effective catalysis. On the other hand, the use of more than 30 % results in the increase in the undesired byproduct as set forth hereinbefore and the decrease in the yield of the polythiobisphenol. Preferably the amount of the catalyst is from 2-20 % by weight, and most preferably from 3-12 % by weight based on the amount of the phenol used. In addition, the use of too much amounts of the catalyst is disadvantageous from a point of view of process economy, for instance, the separation cost for the acid amide hydrochloride produced in large amounts is additionally imposesd on the process as well as the catalyst expenses increase.

In the process of the invention, however, the nitrogen-containing compound as the catalyst may accelerate the elimination of hydrogen chloride generated in the reaction of the phenol and sulfur monochloride.

The use of only a small amount of acid amide catalyst is an important feature of the invention, because substantially no neutralization heat is generated in the reaction, and therefore the reaction can be carried out under milder conditions. For instance, the reaction can be carried out at a much higher and accordingly more practical temperature such as 5 °C to provide the polythiobisphenol in high yields with a minimum

generation of byproducts. Further there is no need to separate the acid amide hydrochloride if generated in the reaction since the amount thereof is very small. These advantages makes the production cost of the polythiobisphenol according to the invention much less expensive compared with the prior process using a large excess amounts of acid amide as a solvent as described hereinbefore.

The second group of the catalyst is a halogen, especially bromine, and an alkali metal halide such as lithium chloride, lithium bromide, lithium iodide or potassium iodide. The catalyst in the second group is normally used in amounts of 200-2000 ppm based on the amount of sulfur monochloride used. The use of less than 200 ppm based on the amount of sulfur monochloride used is insufficient for practical production of polythiobisphenols, while the use of more than 2000 ppm is disadvantageous from a point of view of process economy.

The catalysis of bromine or alkali metal halide may be based on the formation of ClSSX (X: Br or I) by the reaction of sulfur monochloride with MX or $X_2$ (M: an alkali metal such as Li, Na or K; X: a halogen such as Br or I), and the resultant ClSSX reacts with a phenol to form a ClSS-substituted phenol with the simultaneous elimination of hydrogen halide (HX). The hydrogen halide thus formed then reacts with sulfur monochloride to form hydrogen chloride and ClSSX. The reaction scheme is presented below. The bromine or iodine atom in the MX or $X_2$ as the catalyst is likely to be recirculated via HX, i.e., hydrogen bromide or iodide in the reaction system.

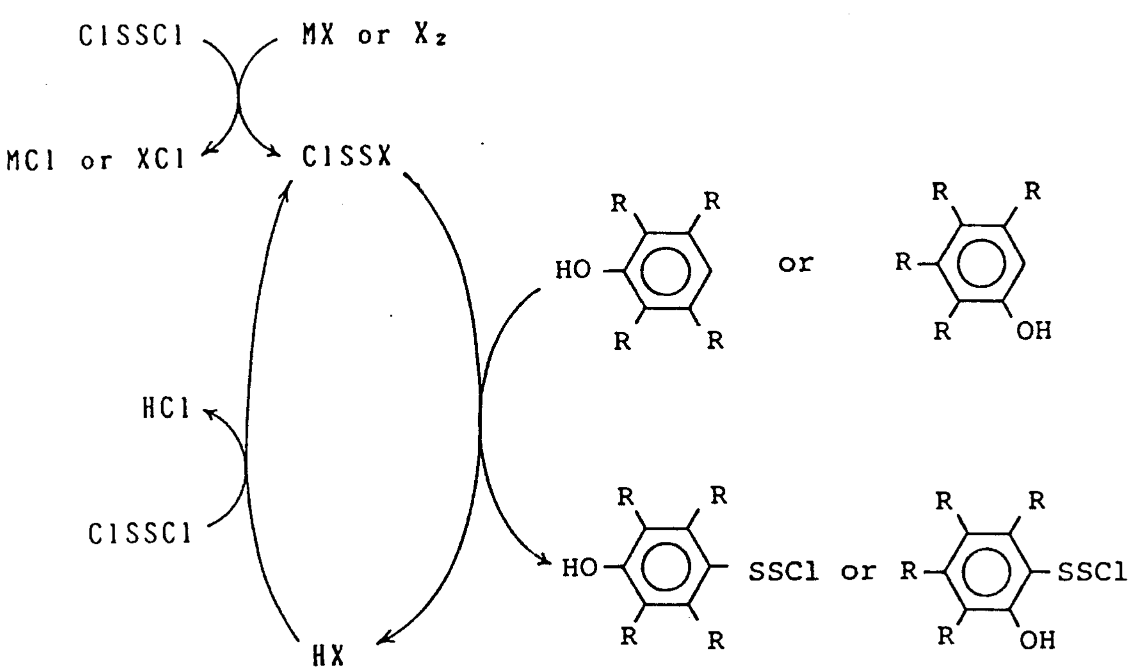

The reaction of the phenol with sulfur monochloride in the presence of the catalyst according to the invention provides the polythiobisphenol having the general formula

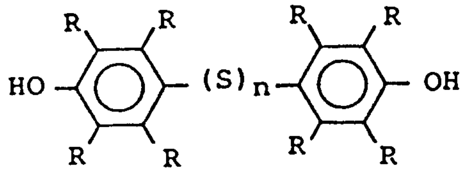 (III)

or

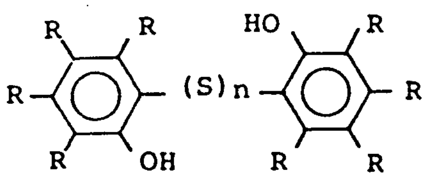

(IV)

wherein R is the same as above, and n is an integer of 2-4.

The main products of the reaction of the invention are di- and/or trithiobisphenols. The selectivity of the reaction is mainly dependent upon the substituents on a phenol aromatic ring. The mechanism of the reaction of the invention is not fully clear, however, when the both sides of the carbon atom as the reaction site are substituted, the main product is trithiobisphenols possibly on account of steric effects concerned.

A preferred embodiment of the present process for producing polythiobisphenols is now presented. At first, 1 mole of phenol is dissolved in an ethylglycol acetate which contains a catalyst therein, for instance, benzyltriethylammonium chloride or pyridine. Then sulfur monochloride in amounts of from 0.5 to 0.75 moles is added dropwise to the solution at temperatures of -20 °C to 50 °C, preferably -20 °C to 30 °C, most preferably -10 °C to 10 °C, while cooling the solution. When the reaction temperature is lower than -20 °C, the reaction proceeds too slowly, and when the reaction temperature is higher than 50 °C, undesired side reactions take place to make the yield of polythiobisphenol small.

The resultant reaction mixture is a yellow or pale yellow homogenious solution, and the polythiobisphenol theren is separable in variuos methods. In a typical method, the solvent is removed by evaporation to leave an oily substance which contains crude polythiobisphenols together with monothiobisphenols as byproducts. Then a nearly the same amount of benzene as the oily substance is added thereto, and the mixture is stirred at the room temperature until the crystallization of the polythiobisphenol begins. Then byproducts soluble in benzene are removed by filtration from the resultant paste-like material to provide polythiobisphenols in yields of 65-90 %. The thus obtained polythiobisphenols may be further purified, when necessary, by recrystallization in benzene or benzene/petroleum ethers.

When the reaction mixture is soluble in water, the reaction mixture after the reaction may be poured into water, the resulting oily substance is separated, and low boiling point components are evaporated at temperatures of 100-110 °C from the oily substance. The crystallization in the same manner as above provides polythiobisphenols. removed from the reaction mixture by filtration. After the toluene is removed from the reaction mixture by distillation, the vacuum distillation of the residue provides 4-mercaptophenol in reduction yields of 90-100 %. When necessary, the rectification by a rectifying column of the product provides highly purified 4-mercaptophenol.

The present invention will be more clearly understood by reference to the following examples, which however are intended only to illustrate the invention and are not to be construed as limiting the scope of the invention.

In the following examples of the production of polythiobisphenols according to the process of the invention, the polythiobisphenol produced was analyzed in the method described below.

## EXAMPLE 1

In a 1000 ml capacity four neck flask provided with a stirrer, a thermometer, a dropping funnel, an inlet tube for nitrogen, a drying tube and a reflux condenser were placed 500 g of ethylglycol acetate. Then 94.1 g (1 mole) of phenol was dissolved in the solvent, and 5 g of N,N-dimethylformamide was added thereto.

The inside of the flask was replaced by nitrogen, and then 74.3 g (0.55 mole) of sulfur monochloride was dropwise added to the mixture with stirring and cooling with ice water to keep the reaction mixture at temperatures of about 5 °C. After the reaction for 4 hours at about 5 °C, the reaction mixture was gradually heated and then the ethylglycol acetate was removed therefrom by vacuum distillation to provide 138.5 g of an oily substance which contained crude polythiobisphenols and monothiobisphenols as byproducts.

A small portion of the oily substance thus obtained is sampled, 20 g of toluene was added thereto and dissolved therein by heating. Then, zinc powder and 35 % hydrochloric acid were added to the mixture and the quantitative reduction of the oily substance was carried out. The resultant organic layer was separated from the reaction mixture, and the resultant mercaptophenol therein was determined by the gas chromathographic internal standard method. Based on this result, the ratio of the phenol initially used to the

amount of the phenol consumed for the production of polythiobisphenols, i.e., the yield of the polythiobisphenol, was found 81 %.

In the same manner as above, phenol was reacted with sulfur monochloride in the presence of N,N-dimethylformamide in varied amounts. The amount of N,N-dimethylformamide used and the resultant yield of polythiobisphenol are shown in Table 1.

TABLE 1

| Amounts of Catalysts (% by weight)[1)] | Yield of Polythiobisphenols (%) |
|---|---|
| 0[2)] | 60 |
| 1.3 | 71 |
| 2.7 | 73 |
| 5.3 | 77 |
| 10.6 | 79 |
| 21.3 | 77 |
| 25.6 | 70 |

1) Based on the weight of phenol used.
2) See Comparative Example 1.

The use of N,N-dimethylacetamide instead of N N-dimethylformamide provided the results similar to those obtained with N,N-dimethylforsamide.

An amount of 50 g of Raney nickel (Kawaken Fine Chemical K.K.) was mixed with 50 g of octyl mercaptane, and the mixture was heated at 100 °C for 2 hours with stirring. Then the water and the mercaptane were removed by vacuum distillation. To the resultant residue was added 50 g of toluene, and then the toluene was removed by filtration from the mixture to provide 38 g of poisoned Raney nickel catalyst.

An amount of 83.1 g of the oily substance which contained the polythiobisphenol (corresponding to 81 % of 0.6 moles of phenol) and 60 g of toluene were placed together with 6.3 g of the poisoned Raney nickel catalyst in a 300 ml capacity autoclave. The inside of the autoclave was replaced by nitrogen, and then the nitrogen was replaced by hydrogen of a pressure of 1.470 $kN/m^2$ (15 $kg/cm^2$). The mixture was then heated to 150 °C, to start the reaction, when the inside was under a pressure of 1.960 $kN/m^2$ (20 $kg/cm^2$). During the reaction, every time the inside hydrogen pressure decreased to 1.764 $kN/m^2$ (18 $kg/cm^2$), hydrogen was supplied into the autoclave to pressurize the inside at 1.960 $kN/m^2$ (20 $kg/cm^2$).

After the reaction at 150 °C for 8 hours when the consumtion of hydrogen in the autoclave ceased, the autoclave was allowed to cool, the pressure inside thereof was released, and the reaction mixture was taken out of the autoclave. The resultant reaction mixture was filtered to remove the catalyst therefrom and then the toluene was removed by vacuum distillation to provide 60.1 g of 4-mercaptophenol (bp. 115-116 °C 666 Pa (5 mmHg)). By gas chromathographic analysis, the 4-mercaptophenol was found 97 % purity. Therefore, the reduction yield calculated was 95 %.

The same Raney nickel as the above were poisoned with various organosulfur compounds in the same manner as above. The hydrogenolysis of polythiobisphenol was carried out in the same manner as above at a temperature of 150 °C. The rate of the hydrogen consumption when the poisoned catalyst was used compared with the hydrogen consumption when the nonpoisoned catalyst was used are shown in Table 2.

TABLE 2

| Organosulfur compounds | Rates of Hydrogen Consumption |
|---|---|
| octyl mercaptane | 2.5 |
| dodecyl mercaptane | 1.9 |
| 2-mercaptoethanol | 1.7 |
| thiophenol | 1.5 |
| 4-mercaptophenol | 1.3 |
| thiophene | 1.3 |
| sulfolane* | 1.0 |
| (nonpoisoned) | 1.0 |

* Sulfolane has no unshared electron pairs.

COMPARATIVE EXAMPLE 1

In the production of polythiobisphenol, the same procedure as EXAMPLE 1 was repeated except that N,N-dimethylformamide was not used. After the addition of sulfur monochloride, an induction period of 2-3 hours was observed, and thereafter the reaction began, and proceeded rather violently. After another 4 hours, the reaction mixture was gradually heated, and then the ethylglycol acetate was removed by vacuum distillation to provide 138.2 g of an oily substance.

The reduction of the oily substance with zinc and hydrochloric acid was carried out, and the reaction product was analyzed in the same manner as in EXAMLE 1. The yield of the polythiobisphenol was found 60 %.

COMPARATIVE EXAMPLE 2

A nickel sulfide hydrogenation catalyst was prepared in a manner suggested by U.S.Patent No. 2,402,614 to Farlow et al.

In a 500 ml capacity four neck flask provided with a stirrer, a thermometer, a dropping funnel, an inlet tube for nitrogen and a reflux condenser were placed 150 ml of water, 24 g of sodium sulfide nonahydrate and 64 g of sulfur, and heated at 50 °C with stirring under nitrogen atmosphere. Almost of the sulfur was found remained undissolved in water.

An aqueous solution of 23.8 g of nickel chloride hexahydrate in 170 ml of water was added dropwise over about 1.5 hours to the above solution of sodium sulfide at a temperature of 50 °C under nitrogen atmosphere. The sulfur became dark but remained undissolved. The mixture was further stirred for another 1 hour.

Thereafter the reaction mixture was filtered, and the solid was twice washed each with 200 ml of water and then thrice each with 100 ml of dioxane, to provide 101 g of paste-like black precipitates. The calculated amount of nickel sulfide in the precipitate was 9.1 g and the remainders were unreacted sulfur, water, etc. The paste-like precipitate was stored until it was used.

In the same manner as in Example 1, 83.1 g of the oily substance as described hereinbefore which contained the polythiobisphenol (corresponding to 81 % of 0.6 moles of phenol) and 60 g of dioxane were placed together with 8.3 g of the paste-like catalyst, and the hydrogenation was carried out at 150 °C for 8 hours under hydrogen of a pressure of 1.960 $kN/m^2$ (20 $kg/cm^2$). The reduction yield of 4-mercaptophenol was calculated to be 12.8 %/0.81, i.e., 15.8 %.

COMPARATIVE EXAMPLE 3

A further nickel sulfide hydrogenation catalyst was prepared in a manner suggested by Farlow et al

An amount of 50 g of the same Raney nickel as used in Example 1 was first washed with methanol and secondly with toluene, and then was immersed in 300 ml of toluene. Then, the Raney nickel in toluene was placed in a 500 ml capacity four neck flask provided with a stirrer, a thermometer, an inlet tube for hydrogen sulfide gas and a reflux condenser. The toluene was refluxed while hydrogen sulfide gas was intoduced into the flask at a rate of 120-160 ml/min. for 2 hours to sulfidate the Raney nickel. The total

amount of hydrogen sulfide introduced was 29 g.

After cooling, nitrogen was bubbled into the reaction mixture to remove the hydrogen sulfide dissolved therein, and then the reaction mixture was washed with toluene until the filtrate bacame colorless and transparent. The resultant sulfidated nickel catalyst was stored in toluene until it was used.

In the same manner as in Example 1, 83.1 g of the oily substance as described hereinbefore which contained the polythiobisphenol (corresponding to 81 % of 0.6 moles of phenol) and 60 g of toluene were placed together with 8.3 g of the catalyst, and the hydrogenation was carried out at 150 °C for 8 hours under a 1.960 kN/m$^2$ (20 kg/cm$^2$) hydrogen pressure. The reduction yield of 4-mercaptophenol was calculated to be 12.4 %/0/81. i.e., 15.3 %.

## COMPARATIVE EXAMPLE 4

This example is to compare the process of British Patent No. 1,345,311 with the process of the invention.

In a 1000 ml capacity four neck flask provided with a stirrer, a thermometer, a dropping funnel, a nitrogen inlet tube, a drying tube and a reflux condenser were placed a solution of 94.1 g (1 mole) of phenol in 400 ml of N,N-dimethylforsamide (401 % by weight based on the phenol).

The inside of the flask was replaced by nitrogen, and then 67.5 g (0.50 mole) of sulfur monochloride in 150 ml of benzene was dropwise added to the mixture over about 4 hours with stirring at a temperature of about -20 °C . After the addition of the sulfur monochloride, the reaction mixture was heated gradually to room temperature, followed by stirring for 2 hours.

A small portion of the reaction mixture was sampled, and was subjected to the quantitative reduction reaction by the use of zinc and hydrochloric acid in the same manner as in Example 1. By quantitative analysis of the resultant mercaptophenol by gas chromathography, the yield of the polythiobisphenol was found 68.7 %.

However, when the reaction was carried out in the same manner as above except the reaction temperature of -5 °C, the yield of the polythiobisphenol was found 61.6 %.

## EXAMPLE 2

In the production of polythiobisphenol, the same procedure as EXAMPLE 1 was repeated except that diethylether and triethylamine were used instead of ethylglycol acetate and N,N-dimethylformamide as a solvent and a catalyst, respectively. 138.6 g of oily substance was obtained. The same analysis as EXAMPLE 1 with this substance proved that the yield of the polythiobisphenol was 73 %.

An amount of 83.2 g of the above oily substance which contained the polythiobisphenol (corresponding to 73 % of 0.6 moles of phenol) and 60 g of toluene were placed together with 6.3 g of the poisoned Raney nickel catalyst prepared in EXAMPLE 1 in a 300 ml capacity autoclave. Then the reaction was carried out in the same manner as in EXAMPLE 1, to provide 54.5 g of 4-mercaptophenol. By gas chromathographic analysis, the 4-mercaptophenol was found 96 % purity. Thus the reduction yield was found 95 %.

## EXAMPLE 3

In the production of polythiobisphenol, the same procedure as EXAMPLE 1 was repeated except that ethyl acetate and pyridine were used instead of ethylglycol acetate and N,N-dimethylformamide as a solvent and a catalyst, respectively. An oily substance was obtained in an amount of 138.1 g. By the same analysis as EXAMPLE 1 with this substance, the yield of the polythiobisphenol was found 76 %.

The hydrogenolysis of 82.9 g of the above oily substance which contained the polythiobisphenol (corresponding to 76 % of 0.6 moles of phenol) with the use of 6.3 g of the poisoned Raney nickel catalyst in the same manner as in EXAMPLE 1 provided 56.5 g of 4-mercaptophenol. The purity was found 97 %, and thus the reduction yield was found 95 %.

## EXAMPLE 4

In the production of polythiobisphenol, the same procedure as EXAMPLE 1 was repeated except that dimethoxyethane and trimethylbenzylammonium chloride were used instead of ethylglycol acetate and N,N-dimethylforsamide as a solvent and a catalyst, respectively. 138.5 g of an oily substance was obtained, The same analysis as in EXAMPLE 1 with this substance showed that the yield of the polythiobisphenol was 80%.

An amount of 20 g of stabilized Raney nickel (Nikko Rika K.K.) was mixed with 20 g of octyl mercaptane, and the mixture was heated at 100°C for 2 hours with stirring. Then the mercaptane were removed by vacuum distillation. To the resultant residue was added 30 g of toluene, and then the toluene was removed by flitration from the mixture to provide 23.5 g of poisoned Raney nickel catalyst.

The hydrogenolysis of 83.1 g of the above oily substance which contained the polythiobisphenol (corresponding to 80 % of 0.6 moles of phenol) was carried out in the same manner as in EXAMPLE 1 at 150°C for 10 hours with the use of 6.3 g of the above poisoned Raney nickel catalyst, to provide 60.2 g of 4-mercaptophenol, which was found 97 % purity. Then, the calculated reduction yield was found 96 %.

EXAMPLE 5

The same procedure as EXAMPLE 1 was repeated except that ethyl acetate and bromine were used instead of ethylglycol acetate and N,N-dimethylformamide as a solvent and a catalyst, respectively. This reaction provided 137.6 g of an oily substance. The same analysis as EXAMPLE 1 with this substance proved that the yield of the polythiobisphenol was 74 %.

The hydrogenolysis of 82.6 g of the above oily substance which contained the polythiobisphenol (corresponding to 74 % of 0.6 moles of phenol) was carried out in the same manner as in EXAMPLE 1 at 150°C for 10 hours with the use of 6.3 g of the poisoned Raney nickel catalyst prepared in EXAMPLE 4, to provide 55.4 g of 4-mercaptophenol, which was found 96 % purity. Thus the reduction yield was found 95 %.

COMPARATIVE EXAMPLE 5

The same procedure as EXAMPLE 5 was repeated except that bromine as a catalyst was not used. After the addition of sulfur monochloride, an induction period of 2-3 hours was observed, and thereafter the reaction began and proceeded rather violently. After another 4 hours, the reaction mixture was gradually heated and then the ethyl acetate was removed by vacuum distillation to provide 137.4 g of an oily substance. The same analysis as EXAMPLE 1 with this substance proved that the yield of the polythiobisphenol was 58 %.

EXAMPLE 6

An amount of 50 g of Raney nickel (Kawaken Fine Chemical K.K.) was mixed with 50 g of dodecyl mercaptane, and the mixture was heated at 100°C for 2 hours with stirring. Then the water and the mercaptane were removed from the mixture by vacuum distillation. To the resultant residue was added 50 g of toluene, and then the toluene was removed by flitration from the mixture to provide 38 g of poisoned Raney nickel catalyst.

The hydrogenolysis of 83.1 g of the oily substance obtained in EXAMPLE 1 which contained the polythiobisphenol (corresponding to 81 % of 0.6 moles of phenol) was carried out in the same manner as in EXAMPLE 1 at 150°C for 10 hours with the use of 6.3 g of the above poisoned Raney nickel catalyst, to provide 60.0 g of 4-mercaptophenol, which was found 97 % purity. Then, the calculated reduction yield was found 95 %.

EXAMPLE 7

An amount of 50 g of Raney nickel (Kawaken Fine Chemical K.K.) was mixed with 50 g of thiophene, and the mixture was heated at 60°C for 3 hours with stirring. After cooling, the mixture was filtered, washed with methanol and then toluene, to provide 39.1 g of poisoned Raney nickel catalyst.

The hydrogenolysis of 83.1 g of the oily substance obtained in EXAMPLE 1 which contained the polythiobisphenol (corresponding to 81 % of 0.6 moles of phenol) was carried out in the same manner as in EXAMPLE 1 at 150°C for 15 hours with the use of 6.3 g of the above poisoned Raney nickel catalyst, to provide 59.2 g of 4-mercaptophenol, which was found 97 % purity. Then, the calculated reduction yield was found 94 %.

EXAMPLE 8

An amount of 83.1 g of the oily substance obtained in EXAMPLE 1 which contained the polythiobisphenol (corresponding to 81 % of 0.6 moles of phenol) and 60 g of isopropanol were placed together

with the 6.3 g of the poisoned Raney nickel catalyst prepared in EXAMPLE 1 in a 300 ml capacity autoclave. The inside of the autoclave was replaced by nitrogen, and then the nitrogen was replaced by hydrogen of a pressure of 1.372 $kN/m^2$ (14 $kg/cm^2$). The autoclave was then heated to 150 °C to start the reaction, when the inside was under a pressure of 1.960 $kN/m^2$ (20 $kg/cm^2$). During the reaction, every time the inside hydrogen pressure decreased to 1.764 $kN/m^2$ (18 $kg/cm^2$), hydrogen was supplied into the autoclave to pressurize the inside at 1.960 $kN/m^2$ (20 $kg/cm^2$).

After the reaction at 150 °C for 14 hours, the autoclave was allowed to cool, the pressure inside thereof was released, and the reaction mixture was taken out of the autoclave. The resultant reaction mixture was filtered to remove the catalyst therefrom and then the isopropanol was removed by distillation followed by vacuum distillation to provide 59.8 g of 4-mercaptophenol. By gas chromathographic analysis, the 4-mercaptophenol was found 97 % purity, and the reduction yield was found 94 %.

EXAMPLE 9

An amount of 83.1 g of the oily substance obtained in EXAMPLE 1 which contained the polythiobisphenol (corresponding to 81 % of 0.6 moles of phenol) and 60 g of dioxane were placed together with 6.3 g of the poisoned Raney nickel catalyst prepared in EXAMPLE 1 in a 300 ml capacity autoclave. The inside of the autoclave was replaced by nitrogen, and then the nitrogen was replaced by hydrogen of a pressure of 1.176 $kN/m^z$ (12 $kg/cm^2$). The autoclave was then heated to 150 °C to start the reaction, when the inside pressure was 1.960 $kN/m^2$ (20 $kg/cm^2$). During the reaction, when the inside pressure decreased to 1.764 $kN/m^2$ (18 $kg/cm^2$), hydrogen was supplied into the autoclave to pressurize the inside at 1.960 $kN/m^2$ (20 $kg/cm^2$).

After the reaction at 150 °C for 11 hours, the autoclave was allowed to cool, the pressure inside thereof was released, and the reaction mixture was taken out of the autoclave. The resultant reaction mixture was filtered to remove the catalyst therefrom and then the dioxane was removed by distillation followed by vacuum distillation to provide 60.0 g of 4-mercaptophenol. By gas chromathographic analysis, the 4-mercaptophenol was found 97 % purity, and the reduction yield was found 95 %.

EXAMPLE 10

An amount of 0.5 moles of a substituted phenol shown in Table 3 was dissolved in 250 g of a solvent shown in Table 3, and was added thereto 2.5 g of N,N-dialkyl acid aside or 0.05 g of bromine or metal halide shown in Table 3 as a catalyst in the same manner as in EXAMPLE 1.

An amount of 37.1-67.5 g (0.27-0.55 moles) of sulfur monochloride was added dropwise to the above solution, and the reaction was carried out in the same manner as in EXAMPLE 1. After the reaction, the solvent was removed by vacuum distillation to provide an oily substance. This substance was sampled, and was reduced in the same manner as in EXAMPLE 1 to determine the yield of the polythiobisphenol by gas chromathography. The results are shown in Table 3.

An amount of the above oily substance which contained polythiobisphenol corresponding to 0.4 to 0.6 moles of phenol and 60 g of toluene were placed together with 6.3 g of poisoned Raney nickel catalyst or 5.3 g of nickel/diatomaceous earth in a 300 ml capacity autoclave, and the hydrogenolysis of the poly

TABLE 3

| Phenols | Solvents[1)] | Catalysts | Moles of $S_2Cl_2$ | Yields of Poly-thiobisphenols (%) | Nickel Catalysts[2)] | Mercaptophenols: Purity[3)] (%) | Mercaptophenols: Reduction Yields (%) | Mercaptophenols: Bp. (℃) |
|---|---|---|---|---|---|---|---|---|
| m-cresol | EGA | DMA | 0.27 | 78 | I | a 96 | 96 | 119-121/(5mmHg) 666 Pa |
| m-cresol | ethyl acatate | LiI | 0.27 | 74 | II | a 95 | 95 | 118-121/(5mmHg) 666 Pa |
| 2,6-xylenol | EGA | DMA | 0.27 | 84 | II | b 96 | 95 | 121-123/(5mmHg) 666 Pa |
| 2,6-xylenol | ethyl acatate | LiBr | 0.27 | 78 | I | b 93 | 95 | 119-123/(5mmHg) 666 Pa |
| 2,6-diisopropyl-phenol | EGA | DMF | 0.41 | 90 | II | c 98 | 95 | 118-120/(5mmHg) 666 Pa |
| 2,6-di-tertiary butylphenol | ethyl acatate | bromine | 0.55 | 88 | II | d 97 | 94 | 125-127/(1mmHg) 133 Pa |
| 2,4-xylenol | EGA | bromine | 0.27 | 72 | II | e 92 | 95 | 92-98/(5mmHg) 666 Pa |

1) EGA: ethylglycol acetate

2) I : poisoned Raney nickel
II : nickel/diatomaceous earth

3) a : 4-mercapto-m-cresol
b : 4-mercapto-2,6-xylenol
c : 4-mercapto-2,6-diisopropylphenol
d : 4-mercapto-2,6-di-tertiary butylphenol
e : 6-mercapto-2,4-xylenol

thiobisphenol was carried out at a temperatures of 150°C for 8-12 hours in the same manner as in EXAMPLE 1. The toluene was removed by distillation followed by vacuum distillation provided mercaptophenols.

The purity of the thus obtained mercaptophenols and the reduction yield calculated as hereinbefore were shown in Table 3.

EXAMPLE 11

An amount of 0.2 moles of a substituted phenol shown in Table 4 was dissolved in 100 g of ethylglycol acetate or ethyl acetate, and was added thereto 2 g of N,N-dimethylformamide or 0.02 g of bromine in the same manner as in EXAMPLE 1.

The reaction was carried out in the same manner as in EXAMPLE 1 by adding 14.9 g (0.11 mole) of sulfur monochloride to the above solution. After the reaction, the solvent was removed by distillation from the reaction mixture.

To the thus obtained oily substance which contained polythiobisphenol was added 80 g of toluene to dissolve the polythiobisphenol therein by the application of heat thereto. An amount of 10 g (0.15 mole) of zinc powder and 35 g (0.34 mole) of 35 % hydrochloric acid were added to the solution to effect the quantitative reduction of the polythiobisphenol. After the reaction, the organic layer was separated from the reaction mixture, made neutral, and washed with water. The toluene was removed by distillation from the organic layer, and then the vacuum distillation of the residue provided the reducion products, i.e., the corresponding mercaptophenols.

The mercaptophenol was subjected to qantitative analysis by gas chromathography to determine the purity thereof and the reduction yield. The results are shown in Table 4.

EXAMPLE 12

Dithiobisphenol was prepared according to the method of Z. S. Ariyan et al. (J. Chem. Soc., 3876 (1962)). An amount of 50.1 g (0.2 mole) of the dithiobisphenol, 60 g of toluene and 5.3 g of nickel/diatomaceous earth catalyst were placed in an autoclave, and the reaction was carried out at 150 °C for 8 hours in the same manner as in EXAMPLE 1. After the reaction, toluene was removed from the reaction mixture followed by vacuum distillation provided 4-mercaptophenol. By gas chromathographic analysis, the 4-mercaptophenol was found to have purity of 98 %, and the reduction yield was found 98 %.

TABLE 4

| Phenols | Solvents[1] | Catalysts | Moles of $S_2Cl_2$ | Products | | | |
|---|---|---|---|---|---|---|---|
| | | | | Mercaptophenols[2] | Purity (%) | Yields (%) | Bp. (℃) |
| o-fluorophenol | EGA | DMF | 0.11 | a | 97 | 72 | 98-100/(5mmHg) 666 Pa |
| o-fluorophenol | ethyl acatate | bromine | 0.11 | a | 95 | 69 | 98-100/(5mmHg) 666 Pa |
| 2-chlorophenol | EGA | DMF | 0.11 | b | 97 | 71 | 95-97/(5mmHg) 666 Pa |

1) EGA: ethylglycol acetate

2) a : 2-hydroxy-5-fluorothiophenol

b : 2-hydroxy-5-chlorothiophenol

## Claims

1. A process for producing polythiobisphenols which comprises: reacting a phenol having the general formula

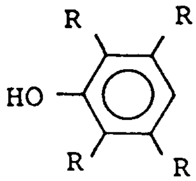

(I)

or

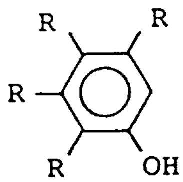

(II)

wherein each R independently represents a hydrogen, a halogen or an alkyl, with sulfur monochloride in a polar organic solvent selected from the group consisting of aliphatic ethers, alicyclic ethers, alkyleneglycol dialkylethers, polyalkyleneglycol dialkylethers, alkyleneglycol monoether monoester, polyalkyleneglycol monoether monoester and aliphatic carboxylic acid alkylesters

in the presence of a nitrogen-containing organic compound as a catalyst which is selected from the group consisting of tertiary amines, quaternary ammoniums, alkylated acid amides and heteroaromatic compounds, in amounts of 1-30 % by weight based on the amount of the phenol used, thereby to provide the polythiobisphenol having the general formula

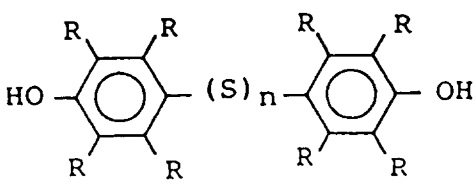

(III)

or

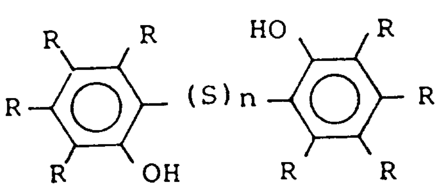

(IV)

wherein R is the same as above, and n is an integer of 2-4.

2. The process according to claim 1 wherein the organic solvent is selected from the group consisting of diethylether, doixane, dimethoxyethane, ethylglycolacetate or ethylacetate.

3. The process as claimed in claim 1 or 2 wherein the tertiary amine is a trialkylamine.

4. The process as claimed in claim 3 wherein the trialkylamine is a triethyl amine.

5. The process as claimed in claim 1 or 2 wherein the quaternary ammonium is a quaternary ammonium chloride.

**6.** The process as claimed in claim 5 wherein the quaternary ammonium chloride is a trialkylaryl ammonium chloride.

**7.** The process as claimed in claim 6 wherein the trialkylaryl ammonium chloride is triethylbenzyl ammonium chloride.

**8.** The process as claimed in claim 5 wherein the quaternary ammonium chloride is a tetraalkyl ammonium chloride.

**9.** The process as claimed in claim 8 wherein the tetraalkylammonium chloride is tetraethyl ammonium chloride.

**10.** The process as claimed in claim 1 or 2 wherein the alkylated acid amide is an N,N-dialkylformamide.

**11.** The process as claimed in claim 10 wherein the N,N-dialkylformamide is N,N-dimethylformamide.

**12.** The process as claimed in claim 10 wherein the alkylated acid amide is an N,N-dialkylacetamide.

**13.** The process as claimed in claim 12 wherein the N,N-dialkylacetamide is N,N-dimethylacetamide.

**14.** The process as claimed in claim 1 or 2 wherein the heteroaromatic compound is pyridine.

**15.** The process as claimed in claim 1 or 2 wherein the catalyst is used in amounts of 2-20 % by weight based on the amount of the phenol used.

**16.** The process as claimed in claim 1 or 2 wherein the catalyst is used in amounts of 3-12 % by weight based on the amount of the phenol used.

**17.** The process as claimed in claim 1 or 2 wherein each R is a hydrogen.

**18.** The process as claimed in claim 1 or 2 wherein the polythiobisphenol is di- and/or trithiobisphenol.

**19.** The process as claimed in claim 1 or 2 wherein the reaction is carried out at temperatures of from -20 °C to 50 °C.

**20.** The process as claimed in claim 1 or 2 wherein the organic polar solvent is used in amounts of 2 to 10 times as much as the weight of the phenol used.

**21.** The process as claimed in claim 20 wherein the organic polar solvent is used in amounts of 3 to 8 times as much as the weight of the phenol used.

**22.** A process for producing polythiobisphenols according to claim 1 characterized by the presence of bromine or an alkali metal halide, in amounts of 200-2000 ppm based on the amount of sulfur monochloride instead of the nitrogen containing compound

**23.** The process as claimed in claim 22 wherein the alkali metal halide is selected from the group consisting of lithium halide, sodium halide and potassium halide.

**24.** The process as claimed in claim 22 wherein each R is a hydrogen.

**25.** The process as claimed in claim 22 wherein the polythiobisphenol is di- and/or trithiobisphenol.

**26.** The process as claimed in claim 22 wherein the reaction is carried out at temperatures of from -20 °C to 50 °C.

**27.** The process as claimed in claim 22 wherein the organic polar solvent is used in amounts of 2 to 10 times as much as the weight of the phenol used.

**28.** The process as claimed in claim 27 wherein the organic polar solvent is used in amounts of 3 to 8 times as much as the weight of the phenol used.

**Patentansprüche**

**1.** Verfahren zur Herstellung von Polythiobisphenolen, umfassend:
Umsetzung eines Phenols der allgemeinen Formel

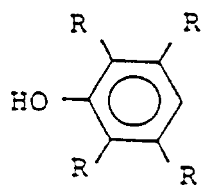

(I)

oder

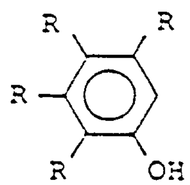

(II)

worin R jeweils unabhängig Wasserstoff, Halogen oder Alkyl bedeutet, mit Schwefelmonochlorid in einem polaren organischen Lösungsmittel, ausgewählt aus der Gruppe bestehend aus aliphatischen Ethern, alicyclischen Ethern, Alkylenglycoldialkylethern, Polyalkylenglycoldialkylethern, Alkylenglycolmonoethermonoestern, Polyalkylenglycolmonoethermonoestern und aliphatischen Carbonsäurealkylestern, in Gegenwart einer Stickstoffhaltigen organischen Verbindung als Katalysator, ausgewählt aus der Gruppe bestehend aus tertiären Aminen, quartärem Ammonium, alkylierten Säureamiden und heteroaromatischen Verbindungen, in Mengen von 1-30 Gew.-%, bezogen auf die Menge des verwendeten Phenols, um so ein Polythiobisphenol der allgemeinen Formel

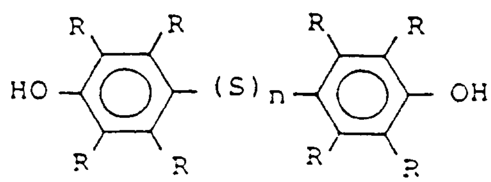

(III)

oder

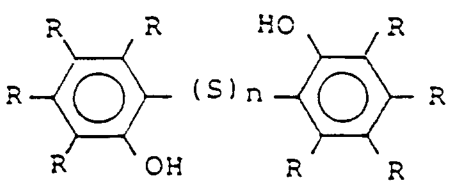

(IV)

bereitzustellen, worin R das gleiche wie oben ist und n eine ganze Zahl von 2-4 ist.

**2.** Verfahren nach Anspruch 1, wobei das organische Lösungsmittel ausgewählt ist aus der Gruppe bestehend aus Diethylether, Dioxan, Dimethoxyethan, Ethylglycolacetat oder Ethylacetat.

3. Verfahren nach Anspruch 1 oder 2, wobei das tertiäre Amin ein Trialkylamin ist.

4. Verfahren nach Anspruch 3, wobei das Trialkylamin Triethylamin ist.

5. Verfahren nach Anspruch 1 oder 2, wobei das quartäre Ammonium ein quartäres Ammoniumchlorid ist.

6. Verfahren nach Anspruch 5, wobei das quartäre Ammoniumchlorid ein Trialkylarylammoniumchlorid ist.

7. Verfahren nach Anspruch 6, wobei das Trialkylarylammoniumchlorid Triethylbenzylammoniumchlorid ist.

8. Verfahren nach Anspruch 5, wobei das quartäre Ammoniumchlorid ein Tetraalkylammoniumchlorid ist.

9. Verfahren nach Anspruch 8, wobei das Tetraalkylammoniumchlorid Tetraethylammoniumchlorid ist.

10. Verfahren nach Anspruch 1 oder 2, wobei das alkylierte Säureamid ein N,N-Dialkylformamid ist.

11. Verfahren nach Anspruch 10, wobei das N,N-Dialkylformamid N,N-Dimethylformamid ist.

12. Verfahren nach Anspruch 10, wobei das alkylierte Säureamid ein N,N-Dialkylacetamid ist.

13. Verfahren nach Anspruch 12, wobei das N,N-Dialkylacetamid N,N-Dimethylacetamid ist.

14. Verfahren nach Anspruch 1 oder 2, wobei die heteroaromatische Verbindung Pyridin ist.

15. Verfahren nach Anspruch 1 oder 2, wobei der Katalysator in Mengen von 2-20 Gew.-% verwendet wird, bezogen auf die Menge des verwendeten Phenols.

16. Verfahren nach Anspruch 1 oder 2, wobei der Katalysator in Mengen von 3-12 Gew.-% verwendet wird, bezogen auf die Menge des verwendeten Phenols.

17. Verfahren nach Anspruch 1 oder 2, wobei R jeweils Wasserstoff ist.

18. Verfahren nach Anspruch 1 oder 2, wobei das Polythiobisphenol Di- und/oder Trithiobisphenol ist.

19. Verfahren nach Anspruch 1 oder 2, wobei die Reaktion bei Temperaturen von -20 °C bis 50 °C durchgeführt wird.

20. Verfahren nach Anspruch 1 oder 2, wobei das organische polare Lösungsmittel in Mengen vom 2- bis 10fachen des Gewichts des verwendeten Phenols verwendet wird.

21. Verfahren nach Anspruch 20, wobei das organische polare Lösungsmittel in Mengen vom 3- bis 8fachen des Gewichts des verwendeten Phenols verwendet wird.

22. Verfahren zur Herstellung von Polythiobisphenolen nach Anspruch 1, dadurch gekennzeichnet, daß anstelle der Stickstoff-haltigen Verbindung Brom oder ein Alkalimetallhalogenid in Mengen von 200-2000 ppm vorhanden ist, bezogen auf die Menge Schwefelmonochlorid.

23. Verfahren nach Anspruch 22, wobei das Alkalimetallhalogenid ausgewählt ist aus der Gruppe bestehend aus Lithiumhalogenid, Natriumhalogenid und Kaliumhalogenid.

24. Verfahren nach Anspruch 22, wobei R jeweils Wasserstoff ist.

25. Verfahren nach Anspruch 22, wobei das Polythiobisphenol Di- und/oder Trithiobisphenol ist.

26. Verfahren nach Anspruch 22, wobei die Reaktion bei Temperaturen von -20 °C bis 50 °C durchgeführt wird.

**27.** Verfahren nach Anspruch 22, wobei das organische polare Lösungsmittel in Mengen vom 2- bis 10fachen des Gewichts des verwendeten Phenols verwendet wird.

**28.** Verfahren nach Anspruch 27, wobei das organische polare Lösungsmittel in Mengen vom 3- bis 8fachen des Gewichts des verwendeten Phenols verwendet wird.

**Revendications**

**1.** Procédé pour produire des polythiobisphénols, qui consiste à faire réagir un phénol ayant la formule générale

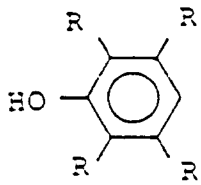

(I)

ou

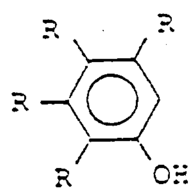

(II)

où chaque radical R, indépendamment des autres, représente un hydrogène, un halogène ou un radical alkyle, avec du monochlorure de soufre dans un solvant organique polaire choisi parmi l'ensemble comprenant les éthers aliphatiques, les éthers alicycliques, les éthers dialkyliques des alkylèneglycols,les éthers dialkyliques des polyalkylèneglycols, le monoester du monoéther des alkylèneglycols, le monoester du monoéther des polyalkylèneglycols et les esters alkyliques des acides carboxyliques aliphatiques,

en présence d'un composé organique azoté servant de catalyseur, qui est choisi parmi l'ensemble comprenant les amines tertiaires, les ammoniums quaternaires, les amides d'acides alkylés et les composés hétéroaromatiques, en des quantités de 1-30 % en poids par rapport à la quantité de phénol utilisée, de façon à obtenir le polythiobisphénol ayant la formule générale

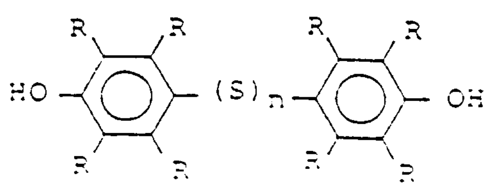

(III)

ou

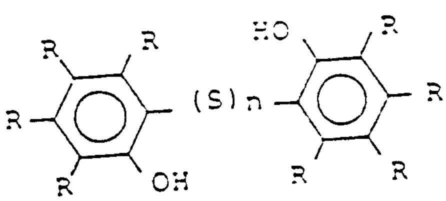

(IV)

dans laquelle R a les mêmes significations que ci-dessus, et n est un entier de 2 à 4.

2. Procédé selon la revendication 1, dans lequel le solvant organique est choisi parmi l'ensemble comprenant le diéthyléther, le dioxanne, le diméthoxyéthane, l'acétate d'éthylglycol et l'acétate d'éthyle.

3. Procédé selon la revendication 1 ou 2, dans lequel l'amine tertiaire est une trialkylamine.

4. Procédé selon la revendication 3, dans lequel la trialkylamine est la triéthylamine.

5. Procédé selon la revendication 1 ou 2, dans lequel l'ammonium quaternaire est un chlorure d'ammonium quaternaire.

6. Procédé selon la revendication 5, dans lequel le chlorure d'ammonium quaternaire est un chlorure de trialkylarylammonium.

7. Procédé selon la revendication 6, dans lequel le chlorure de trialkylarylammonium est le chlorure de triéthylbenzylammonium.

8. Procédé selon la revendication 5, dans lequel le chlorure d'ammonium quaternaire est un chlorure de tétraalkylammonium.

9. Procédé selon la revendication 8, dans lequel le chlorure de tétraalkylammonium est le chlorure de tétraéthylammonium.

10. Procédé selon la revendication 1 ou 2, dans lequel l'amide d'acide alkylé est un N,N-dialkylformamide.

11. Procédé selon la revendication 10, dans lequel le N,N-dialkylformamide est le N,N-diméthylformamide.

12. Procédé selon la revendication 10, dans lequel l'amide d'acide alkylé est un N,N-dialkylacétamide.

13. Procédé selon la revendication 12, dans lequel le N,N-dialkylacétamide est le N,N-diméthylacétamide.

14. Procédé selon la revendication 1 ou 2, dans lequel le composé hétéroaromatique est la pyridine.

15. Procédé selon la revendication 1 ou 2, dans lequel le catalyseur est utilisé en des quantités de 2 à 20 % en poids par rapport à la quantité de phénol utilisée.

16. Procédé selon la revendication 1 ou 2, dans lequel le catalyseur est utilisé en des quantités de 3 à 12 % en poids par rapport à la quantité de phénol utilisé.

17. Procédé selon la revendication 1 ou 2, dans lequel chaque radical R est un hydrogène.

18. Procédé selon la revendication 1 ou 2, dans lequel le polythiobisphénol est le di- et/ou le trithiobisphénol.

19. Procédé selon la revendication 1 ou 2, dans lequel la réaction est mise en oeuvre à des températures de -20 à 50 °C.

20. Procédé selon la revendication 1 ou 2, dans lequel le solvant organique polaire est utilisé en des quantités représentant 2 à 10 fois le poids de phénol utilisé.

21. Procédé selon la revendication 20, dans lequel le solvant organique polaire est utilisé en des quantités représentant 3 à 8 fois le poids de phénol utilisé.

22. Procédé pour produire des polythiobisphénols selon la revendication 1, caractérisé par la présence de brome ou d'un halogénure d'un métal alcalin, en des quantités de 200-2000 ppm par rapport à la quantité de monochlorure de soufre, au lieu du composé azoté.

**23.** Procédé selon la revendication 22, dans lequel l'halogénure de métal alcalin est choisi parmi l'ensemble comprenant les halogénures de lithium, les halogénures de sodium et les halogénures de potassium.

**24.** Procédé selon la revendication 22, dans lequel chaque radical R est un hydrogène.

**25.** Procédé selon la revendication 22, dans lequel le polythiobisphénol est le di- et/ou le trithiobisphénol.

**26.** Procédé selon la revendication 22, dans lequel la réaction est mise en oeuvre à des températures de -20 à 50 °C.

**27.** Procédé selon la revendication 22, dans lequel le solvant organique polaire est utilisé en des quantités représentant 2 à 10 fois le poids de phénol utilisé.

**28.** Procédé selon la revendication 27, dans lequel le solvant organique polaire est utilisé en des quantités représentant 3 à 8 fois le poids de phénol utilisé.